(19)

Europäisches
Patentamt
European
Patent Office
Office européen
des brevets

(11) **EP 1 976 542 B1**

(12) **EUROPEAN PATENT SPECIFICATION**

(45) Date of publication and mention
of the grant of the patent:
**03.03.2010 Bulletin 2010/09**

(51) Int Cl.:
*A61K 36/24* (2006.01)     *A61P 35/00* (2006.01)
*A61P 31/20* (2006.01)     *A61P 31/16* (2006.01)
*A61P 31/18* (2006.01)     *A61P 37/04* (2006.01)
*A61P 29/00* (2006.01)     *A61K 127/00* (2006.01)

(21) Application number: **06792179.1**

(22) Date of filing: **20.09.2006**

(86) International application number:
**PCT/EP2006/009144**

(87) International publication number:
**WO 2007/073785 (05.07.2007 Gazette 2007/27)**

(54) **THERAPEUTIC USE OF AN EXTRACT FROM THE LEAVES OF NERIUM OLEANDER**

THERAPEUTISCHE VERWENDUNG EINES EXTRAKTS AUS DEN BLÄTTERN VON NERIUM OLEANDER

UTILISATION THÉRAPEUTIQUE D'UN EXTRAIT DE FEUILLES DE NERIUM OLEANDER

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **27.12.2005 EP 05028529**

(43) Date of publication of application:
**08.10.2008 Bulletin 2008/41**

(73) Proprietors:
• **Fiebig, Heinz-Herbert**
**79110 Freiburg (DE)**
• **Rashan, Juay Jamil**
**Shafa-Badran 11931 (JO)**

(72) Inventors:
• **FIEBIG, Heinz-Herbert**
**79110 Freiburg (DE)**

• **RASHAN, Juay, Jamil**
**Shafa-Badran 11931 (JO)**
• **RASHAN, Farid, Jamil**
**Mosul (IQ)**

(74) Representative: **Hock, Joachim**
**Müller-Boré & Partner**
**Grafinger Strasse 2**
**81671 München (DE)**

(56) References cited:
**WO-A-02/102395          DE-A1- 3 915 929
NZ-A- 514 794             US-A- 5 135 745
US-A1- 2002 114 852       US-A1- 2005 112 059**

Remarks:
The file contains technical information submitted after the application was filed and not included in this specification

**Description**

The present invention relates to the use of a sterile non-toxic pyrogen-free cold extract from the leaves of *Nerium oleander* as a supplementary medication to cancer chemotherapy to restore and/or ameliorate the immune system of the patient and/or to decrease side effects and increase the antitumor effects of chemotherapeutics, when used in combination with taxol, adriamycin, cisplatin, 5-fluoro-uracil, alimta, cyclophosphamide, mitomycin-C, navelbine, taxotere and topotecan, respectively.

In Arab folk medicine a variety of plants have been used in powder and decoction for treating a number of diseases including cancers and other cell-proliferative and immune deficient diseases. Among these plants are *Peganum harmala, Nerium oleander, Arum spp, Capparis spinosa, Ecballium elatrum* etc. However, the popularity of these plants decreased because of their extreme toxicity both in animals and in humans.

NZ 514794 describes a process for producing a sterile water-soluble extract from *Nerium oleander* containing oleandrin and other digoxin-like glycosides, which is produced by cold extraction in water, for use in the treatment of breast tumors, lung tumors, stomach tumors, colorectal tumors, prostate adenocarcinoma and squamous-cell carcinoma. In addition, there are some publications which describe an extract of plant matter of *Nerium oleander* which is produced by heat extraction (cf. WO 00/16793, EP 0 398 313 A2, EP 0 246 069 B1, WO 02/10239.5 and DE 39 15 929 A1).

Thus, it is the main object underling the present invention to provide a new way for the treatment of cancer or to provide a supplementary medication to cancer chemotherapy. The solution to the above technical problem is achieved by providing the embodiments characterized in the claims.

In particular, there is provided the use of a sterile water-soluble non-toxic pyrogen-free cold extract, obtainable by a method of preparing a cold extract from *Nerium oleander* containing oleandrin and other digoxin-type glycosides comprising the steps of:

(i) soaking a dried powder, preferably about 20 g, obtained from the leaves of *Nerium oleander,* in a sterile medium selected from distilled water, a water/ethanol mixture, a water/methanol mixture, methanol or ethanol, preferably about 100 ml, for a period of about 1 to 50 hours, preferably about 1 to 20 hours, at a temperature in a range of from 0 to 30°C, preferably 0 to 25°C, more preferably 0 to 20°C,

(ii) filtering the resultant solution under sterile-conditions,

(iii) optionally adjusting the volume of the resultant solution to a predetermined volume, preferably about 30 to 40 ml, followed by:

(iv) filtration under sterile conditions, and

(v) optionally spray drying or freeze drying of the filtrate under sterile conditions, as a supplementary medication to cancer chemo-therapy to restore and/or ameliorate the immune system of the patient and/or to decrease side effects and increase the antitumor effects of chemotherapeutics, when used in combination with taxol, adriamycin, cisplatin, 5-fluoro-uracil, alimta, cyclophosphamide, mitomycin-C, navelbine, taxotere and topotecan, respectively. According to the present invention, said cold extract can be used as a supplementary medication to cancer chemo-therapy to restore and/or ameliorate the immune system of the patient and/or to decrease side effects and increase the antitumor effects of chemotherapeutics, when used in combination with taxol, adriamycin, cisplatin, 5-fluoro-uracil, alimta, cyclophosphamide, mitomycin-C, navelbine, taxotere and topotecan, respectively. When used in combination with these commonly known cancer medicaments, no restriction to the kind of cancer to be treated, is given. For example, cancers of bladder, brain, breast, colorectal, head and neck, kidney, liver, lung, ovary, pancreas, pleuramesothelioma, prostate, stomach, testicle, uterus, and vagina as well as Hodgkin's lymphomas, melanomas and sarcomas, can be treated by such a combination therapy.

In the following Tables 1 a and 1 b hereinbelow, the result of the combination studies of the cold extract (also designated as Breastin) used in accordance with the present invention with 5-fluoro-uracil, adriamycin, cisplatin, taxol, alimta, cyclophosphamide, mitomycin-C, navelbine, taxotere and topotecan, respectively, is summarized, where

5FU = 5-fluoro-uracil

ADR = adriamycin

PLAT = cisplatin

TAXOL = taxol

CYACT = cyclophosphamide

RS035 = cold extract according to the present invention (also designated as Breastin)

Accordingly, a pronounced synergism was observed with taxol in 4/6 cell lines, namely the bladder cell line T24, the colon cell line HCT116; the lung line LXF 1121 L and the pancreas cell line PANC1: A synergism was also seen in 2/6 cell lines with adriamycin (T24 and HCT116), in 1/6 cell lines with 5-fluoro-uracil (HCT 116) and in 1/6 cell lines with cisplatin (PANC1); cf. Table 1a. In additional test series, a synergism was also seen with, alimta, cyclophosphamide, mitomycin-C, navelbine, taxotere and topotecan; cf. Table 1b.

**Tab 1A. Combination of Breastin with 4 standard agents in 6 human tumor cell lines**

| Cell line | Exp. no. | Breastin [μg/ml] | IC50 [μg/ml] of standard agent alone or of Breastin In combination with | | | | | | | |
| --- | --- | --- | --- | --- | --- | --- | --- | --- | --- | --- |
| | | | 5-FU | | ADR | | Cis-Platin | | TAXOL | |
| **T24** | GF649 | - | 0,429 | | 0,058 | | >30 | | 0,010 | |
| **(bladder)** | GF649 | 0,1 | 0,401 | - | 0,028 | + | >30 | - | 0,012 | - |
| | GF649 | 0,3 | 0,478 | - | 0,029 | + | >30 | - | 0,003 | ++ |
| **HCT116** | GF650 | - | 0,200 | | 0,006 | | >30 | | 0,0020 | |
| **(colon)** | GF650 | 0,3 | 0,165 | - | 0,006 | - | 19,33 | - | 0,0026 | - |
| | GF650 | 1 | 0,053 | ++ | 0,003 | + | >30 | - | 0,0005 | ++ |
| **LXF 1121L** | GF651 | - | 0,939 | | 0,013 | | >30 | | 0,0018 | |
| **(lung)** | GF651 | 0,03 | 0,625 | | 0,008 | - | >30 | - | 0,0009 | + |
| | GF651 | 0,1 | 0,580 | | 0,010 | - | >30 | - | 0,0009 | + |
| **H460** | GF652 | - | 0,157 | | 0,004 | | 5,26 | | 0,0019 | |
| **(lung)** | GF652 | 0,1 | 0,202 | - | 0,004 | - | >30 | n.e. | 0,0015 | - |
| | GF652 | 0,3 | 0,145 | - | 0,003 | - | >30 | n.e. | 0,0017 | - |
| **MAXF 401NL** | GF653 | - | 0,139 | | 0,007 | | 1,12 | | 0,00044 | |
| **(breast)** | GF653 | 0,1 | 0,300 | - | 0,005 | - | 0,92 | - | 0,0004 | - |
| | GF653 | 0,3 | 0,079 | - | 0,004 | - | 0,88 | - | 0,000 | + |
| **PANC1** | GF654 | - | 0,247 | | 0,022 | | 5,48 | | 0,0019 | |
| **(pancreas)** | GF654 | 0,03 | 0,199 | - | 0,019 | - | 5,48 | - | 0,002 | - |
| | GF654 | 0,1 | 0,185 | - | 0,015 | - | 2,73 | + | 0,0011 | - |
| **Total Synergism** | | | | 1/6 | | 2/6 | | 1/6 | | 4/6 |
| n.e.: not evaluable | | | | | | | | | | |
| Evaluating synergism: | | -, IC70 (combination) >50% of IC70 standard agent alone | | | | | | | | |
| | | +, IC70 (combination) <= 50% of standandard agent alone | | | | | | | | |
| | | ++, IC70 (combination) <= 30% of standard agent alone | | | | | | | | |
| | | +++, IC70 (combination) <= 10% of standard agent alone | | | | | | | | |

| Tab 1B. Combination of Breastin with 6 standard agents in 6 human tumor cell lines | | | | | | | | | | | | | | |
|---|---|---|---|---|---|---|---|---|---|---|---|---|---|---|
| **Cell line** | **Exp.** | **Breastin** | **IC50 [µg/ml] of standard agent alone or of Breastin in combination with** | | | | | | | | | | | |
| | **no.** | **[µg/ml]** | **Alimta** | | **CYACT** | | **Mitomycin** | | **Navelbine[1]** | | **Taxotere[1]** | | **Topotecan** | |
| **T24** | GF693 | - | 0,0010 | | 0,320 | | 0,056 | | 0,144 | | 0,163 | | 0,0032 | |
| **(bladder)** | GF693 | 0,3 | 0,0018 | - | 0,490 | | 0,056 | - | 0,173 | - | 0,187 | - | 0,0035 | - |
| | GF693 | 1 | 0,0026 | - | 0,586 | | 0,042 | - | 0,162 | ++ | 0,123 | - | 0,0041 | - |
| **HCT116** | GF681 | - | 0,021 | | 0,515 | | 0,022 | | 0,060 | | 0,035 | | 0,0066 | |
| **(colon)** | GF681 | 0,3 | 0,016 | - | 0,716 | - | 0,025 | - | 0,066 | - | 0,065 | - | 0,0060 | - |
| | GF681 | 1 | 0,008 | + | 0,069 | ++ | 0,002 | + | 0,043 | - | 0,020 | - | 0,0045 | - |
| **LXF 1121L** | GF694 | | 0,014 | | 1,90[2] | | 0,055 | | 0,0050 | | 0,079 | | 0,0050 | |
| **(lung)** | GF694 | 0,1 | 0,012 | - | 0,88[2] | + | 0,043 | - | 0,0034 | - | 0,017 | ++ | 0,0026 | - |
| | GF694 | 0,3 | 0,028 | - | 1,00[2] | - | 0,032 | - | 0,0041 | - | 0,003 | ++ + | 0,0027 | - |
| **H460** | GF695 | | 0,018 | | 0,495 | | 0,009 | | 0,057 | | 0,059 | | 0,0079 | - |
| **(lung)** | GF695 | 0,3 | 0,016 | - | 0,243 | + | 0,006 | - | 0,051 | - | 0,057 | - | 0,0067 | - |
| **MAXF 401NL** | GF682 | | >100 | | 0,121 | | 0,048 | | 0,0083 | | 0,024 | | 0,0017 | |
| **(breast)** | GF682 | 0,3 | 0,035 | +++ | 0,065 | - | 0,032 | - | 0,0055 | - | 0,011 | + | 0,0018 | - |
| | GF682 | 1 | n.e. | | 0,002 | +++ | 0,0003 | +++ | 0,0022 | ++ | 0,004 | ++ | 0,0002 | ++ |
| **PANC1** | GF683 | | >100[2] | | 0,193 | | 0,228 | | 0,119 | | 0,153 | | 0,0055 | |

(continued)

| Cell line | Exp. no. | Breastin [µg/ml] | IC50 [µg/ml] of standard agent alone or of Breastin in combination with | | | | | | | | | | | |
|---|---|---|---|---|---|---|---|---|---|---|---|---|---|---|
| | | | Alimta | | CYACT | | Mitomycin | | Navelbine[1] | | Taxotere[1] | | Topotecan | |
| (pancreas) | GF683 | 0,1 | >100[2] | - | 0,235 | - | 0,116 | - | 0,128 | - | 0,219 | - | 0,0025 | + |
| | GF683 | 0,3 | >100[2] | - | 0,147 | - | 0,092 | + | 0,120 | - | 0,064 | + | 0,0023 | + |
| | | | | | | | | | | | | | | |
| Total synergism | | | 2/6 | | 4/6 | | 3/6 | | 1/6 | | 3/6 | | 2/6 | |
| | | | | | | | | | | | | | | |
| [1]IC50 values given in µg/ml | | | | | [2]based on IC70 values | | | n.e not evaluable | | | | | | |
| Evaluating synergism: | | | | | -, IC70 (combination) >50% of IC70 standard agent alone | | | | | | | | | |
| | | | | | +, IC70 (combination) <= 50% of standandard agent alone | | | | | | | | | |
| | | | | | ++, IC70 (combination) <= 30% of standard agent alone | | | | | | | | | |
| | | | | | +++, IC70 (combination) <= 10% of standard agent alone | | | | | | | | | |

The above-mentioned method of preparing a cold extract of *Nerium oleander* is an easily reproducible one-step process for preparing a sterile water-soluble non-toxic pyrogen-free cold extract from the leaves of *Nerium oleander.* The water-soluble cold extract of *Nerium oleander* can be used in the form of a sterile aqueous extract solution (produced by using steps (i) to (iv)) or in the form of a lyophilized (freeze-dried) powder (produced by using steps (i) to (v)) and, if desired, together with a commonly used pharmaceutically acceptable carrier and optionally one or more additives. This cold extract is non-toxic, pyrogen-free and directed to antioncogenic, antiviral and immunostimulating activity.

The method of drying the *Nerium oleander* leaves is not particularly restricted and it is possible to dry the leaves at room temperature or at slightly elevated temperatures, e.g. by air drying. However, the dried powder to be used in step (i) is preferably produced by immediately subjecting the conected *Nerium oleander* leaves to a drying treatment at a temperature of 20 to 50°C. Preferably, the collected *Nerium oleander* leaves are dried at a temperature of about 40°C in a drying oven for 48 to 96 hours, preferably 72 hours. Subsequently, the dried *Nerium oleander* leaves are ground in an electrical grinder to obtain a fine powder having a particle size of e.g. about 1 to 10 $\mu$m.

The active ingredients of *Nerium oleander* are preferably extracted from the leaves of *Nerium oleander* having a length of 16 to 19 cm which are harvested during a period of from August to December, most preferably in October.

In the above-mentioned method of preparing a cold extract from *Nerium oleander,* step (i) is preferably conducted at about room temperature and under sterile conditions.

In step (iv) it is preferred to use a filter having a pore size in a range of 0.22 to 0.45 $\mu$m, most preferably a Millipore® filtration system having a pore size of 0.45 and/or 0.22 $\mu$m. According to a preferred embodiment of the present invention, a Millipore® filtration having a pore size of 0.45 $\mu$m is used, followed by a Millipore® filtration having a pore size of 0.22 $\mu$m.

The above-mentioned method of preparing a cold extract from *Nerium oleander* is suited to achieve a sterile extract with endotoxin concentrations of less than 30 units/ml.

The cold extract used in the course of the present invention preferably includes oleandrins, oleandrigenin as major glycosides with trace amounts of digitoxin, gitoxigenin, flavonoids, rutin and triterpenoides, sugar such as xylose, galactose, glucose, mannose and arabinose, and polysaccharides such as D-galacto-uronic acid, and amino acid moieties. For example, in 125 mg of a dry extract according to the present invention 0.23 mg oleandrin and 0.19 mg oleandrigenin are contained. Other glycosides are present in minor amounts.

Usually; wild *Neriuim oleander* plants are harvested in a selected area close to running water.

According to a preferred embodiment of the present invention the extract is obtainable by:

(a) rinsing the ground powder of *Nerium oleander* in a sterile medium selected from distilled water, a water/ethanol mixture, a water/methanol mixture, methanol or ethanol, (preferably 1/5 w/v), for 4 to hours at a temperature in a range of 0 to 30°C, preferably 0 to 25 °C, more preferably 0 to 20 °C, and
(b) filtering the obtained suspension several times and adjusting the pH value to a range of 5.7 to 6.0 under laminar flow conditions using a filter having a pore size in a range of 0.22 to 0.45 $\mu$m, preferably a Millipore® filtration system having a pore size of 0.45 $\mu$m and/or a Millipore® filtration system 0.22 $\mu$m.

Preferably, (c) the filtered suspension of step (b) may be filtered by using a Millipore® filtration system having a pore size of 0.45 $\mu$m and, then, a Millipore® filtration system having a pore size of 0.22 $\mu$m under laminar flow conditions, wherein (d) the concentration of endotoxins in the suspension of step (c) is tested and adjusted to less than about 30 units/ml and (e) no bacterial growth in the suspension of step (d) is determined after 48 hours of incubation.

The above-mentioned sterile water-soluble cold extract has both *in vitro* and *in vivo* biological characteristics, wherein the *in vitro* characteristics include:

(a) cytotoxic activity against Hela, $K_{562}$, Raji, KB, Hep-2 ($IC_{50}$ from 1-5 $\mu$g/ml) and a moderate cytotoxicity against $P_{388}$, $L_{1210}$, $T_{47}D$, RD, $A_{549}$, $L_{20}B$ ($IC_{50}$ 6-10 $\mu$g/ml),
(b) significant anticancer activity against 31 out of 63 human permanent cell lines, established from bladder, liver, ovary, pancreas, testicle and uterus carcinoma as well as sarcomas and further from brain, colon, head and neck, lung, mammary, melanomas, prostate, and renal carcinoma as well as sarcomas ($IC_{50}$: 0.09 to 1.14 $\mu$g/ml),
(c) significant anticancer activity against 24 out of 67 patient derived human tumors established in nude mice and studied in the tumor stem cell assay activity was observed in bladder, ovarian, pancreas, pleuramesothelioma, and uterus cancers and further also in brain, colon, lung, mammary, prostate, and renal cancers as well as in melanomas and sarcomas,
(d) hematopoetic stem cells of 3 human donors were less sensitive,
(e) a block at the S-phase of the cell cycle at a concentration of 1 $\mu$g/ml for $K_{562}$ and for $P_{388}$ and KB at 10 $\mu$g/ml,
(f) a significant ribosome-inactivating protein activity (RIP) at concentrations of less tan 1 $\mu$g/ml,
(g) no mutagenic effects against two bacterial strains: S. typhimurium $TA_{1530}$ and S. typhimurium $TA_{1537}$ at concentrations of up to 50 $\mu$g/ml,

(h) release of the cytokines IL-6, TNF-$\alpha$, IL-1b from PBMC's indicating stimulation of macrophages/monocytes, NK-cells and B-lymphocytes,

(i) IFN-production in whole blood *in vitro,*

(j) very significant anticomplementary effect at concentrations of less than 1 $\mu$g/ml *in vitro,*

(k) immune-modulatory effect to sheep red blood cells and to P-815 mastocytoma cell lines *in vitro* ($IC_{50}$: 0.31 and 2.5 $\mu$g/ml, respectively),

(l) inhibition of influenza viruses replication A/Texas/77; A/Philippines/81 and B/Hong Kong/79 *in vitro* with an $IC_{50}$ = 0.3 $\mu$g/ml,

(m) activity against HIV-1 at concentrations of less than 1 $\mu$g/ml and against Poliovirus type I (Sb1),

(n) significant inhibition of the reverse-transcriptase enzyme *in vitro* ($IC_{50}$: 4 to 5 $\mu$g/ml), and

(o) no antiviral activity against Herpes virus simplex type I (HSV-1), Reovirus (Reo) and Yellow fever virus (YFV) *in vitro* at concentrations of up to 20 $\mu$g/ml.

The *in vivo* characteristics of said sterile water-soluble cold extract include:

(a) a significant inhibition of tumor growth of B16-Melanoma in $BDF_1$ mice (T/C = 135 % at the dose of 0.025 ml per 25 g mouse), and a significant inhibition of tumor growth of Lewis lung carcinoma in $DBF_1$ mice (T/C = 127 % and 137 % at the doses of 0.025 ml and 0.05 ml respectively),

(b) no activity against leukaemia models $P_{388}$ and $L_{1210}$ (T/C = 109 % and 104 % respectively for the dose of 0.05 ml),

(c) no cutaneous effects on guinea pig skin and no inflammatory changes in the eyes of rabbits at the dose of 0.5 ml and 0.1 ml respectively,

(d) a $LD_{50}$ in mice is 0.35 ml/25 g IM and 0.3 ml and 0.33 ml/25 g IP and SC, respectively, with apparent side effects, at high doses, like tachycardia, myorelaxation and motoric incoordination,

(e) no apparent side effects at doses up to 0.1 ml/25 g in single or repeated doses,

(f) a significant increase in leukocytes count (mostly in lymphocytes) at a dose of 0.05 ml, when studied subchronically for 8 weeks in mice; when studied chronically for 6 months in rats, the extract causes no significant difference in the kidney function tests, but significant increase was observed in the AST and ALP enzymes in the treated groups compared to untreated controls,

(g) no effect on body weight, hemoglobin concentration, erythrocyte count and blood indices like MCV, HCH and MCHC subchronically for a period of 8 weeks in mice, and chronically for a period of 6 months in rats,

(h) no gross-abnormalities in the organs of treated groups when studied chronically for 6 months in rats,

(i) significant myorelaxation activity in mice at concentrations of 0.15 to 0.3 ml/25 g, and

(j) a dose-related sedative effect in mice and a depression of all behaviour aspects at 0.3 ml/25 g.

The sterile water-soluble cold extract can be used for the intramuscular, oral, rectal, and intravenous administration.

In a further embodiment, the sterile water-soluble non-toxic pyrogen-free extract, obtainable by a method of preparing a cold extract from *Nerium oleander* containing oleandrin and other digoxin-type glycosides as described above, will also be used in viral, infectious and inflammatory diseases such as hepatitis B, hepatitis C, influenza, and immune deficient disorders like HIV/AIDS.

In still a further embodiment, the sterile water-soluble non-toxic pyrogen-free extract, obtainable by a method of preparing a cold extract from *Nerium oleander* containing oleandrin and other digoxin-type glycosides as described above, will be used as a food supplement.

From the above-mentioned sterile cold extract of *Nerium oleander* a medicament or a pharmaceutical composition can be produced with at least one pharmaceutically acceptable carrier. Preferably, the pharmaceutical composition is suited for injection. Alternatively the pharmaceutical composition may be in the form of an oral or rectal formulation or a topical preparation.

Preferably, the pharmaceutical composition is effective in treating the above-mentioned disorders or diseases, e.g. human tumors, wherein 0.2 to 0.4 ml (2 to 4 mg) are injected by intramuscular route on daily basis followed by an oral maintenance therapy.

Alternatively, 2 to 4 mg of the pharmaceutical composition can be injected by intramuscular route once or twice a week.

The pharmaceutical composition may also be in the form of an oral or a rectal formulation and topical preparation, wherein 7 to 10 mg are given daily.

The treatment of *Nerium oleander* leaves with water, a mixture of water and ethanol or methanol, ethanol or methanol gives a full range of extracts having antitumor, antiviral, immunmodulatory and cardiotonic activities. The extracts, upon evaporation to dryness, provide a solid residue, characterised by a complex physico-chemical fingerprint.

It should be stressed that the cold extract used in accordance with the present invention is really different from the hot extract. Not only the preparation of the cold extract is quite different from that of a respective hot extract, but also the composition is quite different. While the cold extract is obtained by one-step procedure, the hot extract is prepared by many-steps that involves heating, etc., as can be taken for example from DE 39 15 929 A1. When the two extracts are analysed using different chemical techniques such as HPLC, LC/MS, high resolution LC/MS, LC/MS/MS, and high resolution NMR, it is evident from the scans that:

(1) The cold extract contains more glycosides when compared to the hot extract and this can be attributed to the fact that during boiling the sugars will cleave off and become free, whereas during cold extraction the sugars become connected to the glycosides.

(2) The results of the above analysis also indicated the presence of triglycosides in the cold extract, whereas monoglycosides were detected in the hot extract. This can be proven by the respective high resolution LC/MS chromatograms. Furthermore, the respective two different preparations show a different antitumor activity. The cold and the hot extract have been compared in 36 human tumor/cell lines. The cold extract (also designated as Breastin) showed a higher antitumor activity and tumor selectivity than the hot extract. The mean inhibitory concentration IC70 was quite lower for Breastin (2.025 $\mu$g/ml) *versus* 12.994 $\mu$g/ml for the hot extract indicating that the cold extract is 6.4 times more active with a more pronounced tumor selectivity. Therefore, it is evident that the different compositions also are responsible for the much higher antitumor activity of the cold extract. Details are shown in Table 2 hereinbelow.

**Table 2**

| IN-VITRO ANTITUMOR ACTIVITY OF RS035 (Cold Extract) and RS034 (Hot Extract | | | |
|---|---|---|---|
| TUMOR/ | EXP. | Cold Extract | Hot Extract |
| PASSAGE NO. | NO. | IC70 ug/ml | IC70 ug/ml |
| **BXF, Bladder** | | | |
| 1218L | *(2) | 1.781 | 14.724 |
| T24 | *(2) | 1.029 | 9.486 |
| | | | |
| **CNXF, Central Nervous System** | | | |
| 498NL | * (2) | 1.773 | 12.986 |
| SF268 | *(2) | 0.251 | 2.619 |
| | | | |
| **CXF, Colon** | | | |
| HCT116 | *(2) | 1.447 | 12.276 |
| HT29 | *(2) | 1.798 | 16.295 |
| | | | |
| GXF, Gastric | | | |
| 251L L | *(2) | 12.570 | 27.425 |
| **HNXF, Head& Neck** | | | |
| 536L | *(2) | >30.000 | >30.000 |
| | | | |
| **LXF, Lung NSCLC** | | | |
| 1121L | *(2) | 0.190 | 2.093 |
| 289L | *(2) | 2.027 | 19.886 |
| 526L | *(2) | 1.514 | 12.410 |
| 529L | *(2) | 0.898 | 6.027 |
| 629L | *(2) | 1.973 | 16.139 |
| H460 | *(2) | 0.884 | 6.340 |
| **MAXF, Mammary** | | | |
| 401NL | * (2) | 1.852 | 19.043 |
| MCF7 | * (2) | 1.594 | 10.048 |

(continued)

| IN-VITRO ANTITUMOR ACTIVITY OF RS035 (Cold Extract) and RS034 (Hot Extract | | | |
|---|---|---|---|
| TUMOR/ | EXP. | Cold Extract | Hot Extract |
| PASSAGE NO. | NO. | IC70 ug/ml | IC70 ug/ml |
| **MEXF, Melanoma** | | | |
| 276L | * (2) | 12.373 | 32.571 |
| 394NL | * (2) | 3.302 | 18.185 |
| 462NL | * (2) | 1.729 | 15.944 |
| 514L | *(2) | 2.085 | 21.089 |
| 520L | * (2) | 2.054 | 15.304 |
| **OVXF, Ovary** | | | |
| 1619L | FG7431T | >30.000 | >30.000 |
| 899L | * (2) | 2.017 | 19.324 |
| OVCAR3 | * (2) | 1.255 | 17.054 |
| **PAXF, Pancreas** | | | |
| 1657L | * (2) | 2.116 | 16.399 |
| PANC1 | * (2) | 0.440 | 3.806 |
| **PRXF, Prostate** | | | |
| 22RV1 | * (2) | 1.911 | 15.096 |
| DU145 | *(2) | 0.558 | 2.330 |
| LNCAP | * (2) | 1.792 | 18.549 |
| PC3M | *(2) | 1.623 | 10.781 |
| **PXF, Pleuramesothelioma** | | | |
| 1752L | * (2) | 78.790 | 73.027 |
| **RXF, renal** | | | |
| 1781L | *(2) | 1.524 | 6.185 |
| 393NL | * (2) | 1.439 | 14.771 |
| 486L | * (2) | 1.620 | 13.025 |
| 944L | * (2) | 1.449 | 14.549 |
| **UXF, Uterus** | | | |
| 1138L | * (2) | 1.826 | 15.614 |
| Mean | n=36 | 2.025 | 12.994 |

Accordingly, it is evident that a *Nerium oleander* extract which is produced by heat extraction results in a different extract having less activity when used in a pharmaceutical composition compared to a respective cold extract in preclinical cancer models. For example, a cold extract contains more glycosides and flavonoids, but less polysaccharides than a hot extract.

The following examples are given by way of illustration only and are not limiting the scope of the invention. In the following, the cold extract used in accordance with the present invention is also designated as Breastin.

**Examples**

**Preparation of a *Nerium oleander* extract and its characterization**

A. Preparation of a *Nerium oleander* cold extract:

200 g of sterile dried, finely ground leaves obtained from *Nerium oleander* are suspended in 1000 ml of distilled water and are stirred at room temperature (i.e. 22-23°C) under laminar flow conditions. The solution was filtered several times using Whatmann no. 1 filter paper and Millipore® filtrations. The volume of the clear, dark brown filtrate is adjusted to 350 ml and the pH value of the solution is adjusted to 5.9. The filtrate was divided into two equal portions. The first portion was aliquoted under laminar flow conditions into sterile vials and some were stored at 4°C in the refrigerator and the others were left at room temperature conditions. The second portion of the extract was lyophilized (freeze-dried) under sterile conditions and the dry powder was stored in sterile containers.

B. Sterility Tests:

The following tests were applied:

(1) direct swab culture using different media and direct staining smear technique after 48 hours of incubation,
(2) determination of the concentration of bacterial endotoxins in the extracts and the powder using the conventional Limulus Amoebocyte Lysate (LAL) test, and
(3) the pyrogen test of the extract of the lyophilized powder using rabbits.

No bacterial growth was observed after 48 hours of incubation of the extract and the lyophilized powder with different growth media. Furthermore, the concentration of bacterial endotoxins was less than 30 units/ml. However, the pyrogen tests in rabbits showed an increase in the temperature at about 2 to 2.30 hours after intravenous administration.

C. Physico-Chemical Characterisation

100 ml of the aqueous extract are evaporated to dryness at 25°C under vacuum. The solid extract is kept under vacuum in a desiccator overnight. Aliquots from both the solid residue and the lyophilized fraction are used for TLC and sugar and amino acid tests.

**1) Cardiac Glycosides:**

The presence of glycosides in the room temperature *Nerium oleander* extract was examined by the following TLC method, which showed positive results and were compared with the standard glycosides.

(i) Plate: Silica gel G, 250 nm thick, activated at 120°C for 45 min,
(ii) Mobile phase: benzene : ethanol (7:3),
(iii) Location Reagent: p-anisaldehyde reagent prepared by mixing 10 ml of anisaldehyde, 90 ml of ethanol and 10 ml of concentrated $H_2SO_4$.

Sample: The solid (100 mg) was suspended in 1 ml of a mixture of $CHCl_3$ and methanol (1:1) with gentle heating on water bath, and then centrifuged.
The clear pale yellow solution was placed on the silica gel plate. Comparative samples of the standards (0.2 mg/ml) were prepared using the same solvent and 100 $\mu$l of the resulting solution was placed onto the plate.

Procedure and Results

The usual procedure for TLC was carried out. After spraying the reagent, the plate was heated in an oven at 100°C for 4 min. The spots appeared were varied in color from yellow, blue, violet, green and olive green and with the following $R_f$ values:
0.16, 0.23, 0.29, 0.40, 0.45, 0.51, 0.55, 0.60, 0.61, 0.66, 0.70, 0.73.
The $R_f$ values obtained from the standards are as follows:

| Standards | $R_f$ Values |
|---|---|
| Oleandrin | 0.69 |
| Oleandrigenin | 0.65 |
| Digitoxin | 0.59 |
| Gitoxigenin | 0.56 |
| Digoxin | 0.51 |
| Stigmasterol | 0.77 |

Therefore, in general, the $R_f$ values of the glycosides present in the extract are well comparable with those of the standards.

2) Sugar Test:

A positive test was obtained on using Fehling's solution reagent, as follows: 50 mg of the solid and the lyophilized fractions were boiled with 0.1 N HCl (5 ml) for about 1 hour. The acidic solution was then neutralised by addition of sodium hydroxide (0.5 N). The total volume was adjusted to about 3 ml by heat evaporation. To this was added a solution of Fehling 1 and 2 (3 ml each) and distilled water (18 ml) and the mixture was heated to the boiling point for 10 minutes. The color turned olive-green with the precipitation of a red solid indicating the presence of sugar moiety in both fractions, due to the formation of $Cu_2O$.

3) Amino Acids Test:

A positive test was obtained for amino acids on using the ninhydrin solution reagent (0.25 g) in acetone (20 ml), as follows:

Filter Paper Method:

The solid and lyophilized fractions were dissolved in a mixture of water and ethanol (1:2) and one drop of the resulting solution was placed onto filter papers and one drop of the reagent was added. The filter papers were dried over a stream of hot air. A violet color immediately appeared indicating the presence of amino acids in both fractions.

Solution Method:

The solid (50 mg) from both fractions was dissolved in 5 ml water and 3 ml of the reagent was added. The reaction mixture was heated with continuous stirring for about 15 minutes. A deep violet color solution was observed from both fractions indicating the presence of amino acid moieties.

4) Polysaccharides

D-galacto-uronic acid was detected.

5) More detailed analysis

The following compounds are isolated in a pure form from the cold aqueous extract of *Nerium oleander.*

    1. odoroside H [digitoxigenin digitaloside] Mt = 534
    2. odoroside A [digitoxigenin diginoside] Mt =518
    3. oleandrin [oleandrigenin-oleandroside] Mt = 576
    4. oleandrigenin sarmentoside Mt = 576
    5. oleandrigenin diginoside Mt = 576
    6. neritaloside (oleandrigenin digitaloside) Mt = 592
    7. oleandrigenin-B-D-glucopyranoside Mt = 594
    8. 16-androdigitoxigenin-digitaloside Mt =532
    9. 16-anhydrodigitoxigenin sarmentoside Mt = 516
    10. 16-anhydrodigitoxigenin diginoside Mt = 516
    11. vanderoside Mt =534

12. adynerine Mt =516

13. 3-0-digitalosyl-8, 14-epoxy-3-hydroxy-carda 20 (22)-enolide Mt = 532

14. 3-0-diginosyl-8, 14-epoxy-3-hydroxy-carda 16, 20 (22)-dienolide Mt = 514

15. 3-0-sarmentosyl-8, 14-epoxy-3-hydroxy-carda-16, 20 (22) dienolide Mt =514 (which is a new compound)

16. 3-0-digitalosyl-8, 14-epoxy-3-hydroxy-carda 16; 20 (22)-dienolide

17. rutin Mt = 510 (separated in large quantities by silica gel, more than 63 mg) Some blumenol derivatives plus hexose (glucose) and pentose (xylose, apiose, arabinose) were also isolated in the impure forms and such compounds were not detected previously in *Nerium oleander.*

6) Conclusion

The cold extract of *Nerium oleander* was investigated using different chemical techniques including TLC, HPLC, column-chromatographic separation techniques, LC/MS, high resolution mass and LC/MS, LC/MS/MS and high resolution H-NMR.

A number of already known compounds in oleander was isolated in a pure form such as oleandrin, oleandrigenin, adynerin, vanderoside, rutin, etc. On the other hand, the new compound "3-0-sarmentosyl-8, 14-epoxy-3-hydroxy-carda-16, 20 (22)-dienolide" having a molecular weight of 514 was isolated. In addition, for the first time the presence of oleandrigenin-B-D-glucopyranoside has been observed, which according to literature was detected in *Nerium odorum* and *adenium obesum* but not in *Nerium oleander.* Finally, the cold extract also contains some blumenol derivatives plus hexose (glucose) and pentose (xylose, apiose, arabinose) and such compounds are not detected before in oleander.

According to the results of antitumor activities performed on these compounds as described above, this activity can be attributed mainly to oleandrin; oleandrigenin; odoroside A, H; vanderoside; adynerine; 16-anhydrodigitoxigenin-diginoside ($IC_{70} > 1$ ug/mL) and to the blumenol derivatives ($IC_{70}$ 1-3 ug/mL) and also to some extent to the 3-0-sarmentosyl-8, 14-epoxy-3-hydroxy-carda-16, 20 (22) dienolide ($IC_{70}$ 6.5 ug/mL). Some activity was also detected in the polysaccharide fraction isolated from the oleander ($IC_{70} < 10 \mu g/ml$)

### Injectable preparation

Breastin as an injectable preparation contains the substances as analyzed above, in particular: oleandrin, oleandrigenin and digitoxigenin as major compounds and other digoxin-type glycosides, flavonoids, rutin and triterpenoides, simple sugars such as xylose, galactose, glucose, mannose and arabinose, and polysaccharides such as D-galacto-uronic acid, and amino acid moieties.

### Oral preparation

As above plus starch.

### Preclinical Pharmacology

A. Anticancer activity *in vitro:*

1. In human cell lines *in vitro*

The anticancer activity of an oral preparation comprising the cold extract according to the present invention (designated as Breastin in the following) was investigated as follows:

The anticancer properties of Breastin were tested in 36 human cancer cell lines in a monolayer assay after 4 days incubation time. 5.000 to 10.000 cells were plated per well in 96 wells, Breastin was added 1 day later and left over for 4 days. An equivalent of cell number was determined with the fluorescence dye propidium iodide. Inhibition of cell number under therapy compared to the control was taken for evaluation. Inhibitory concentrations 50, 70 and 90 were calculated.

Breastin showed a high anticancer activity in 31 out of 63 human cancer cell lines derived from bladder, kidney, liver, ovary, pancreas, testicle and uterus as well as pleuramesotheliomas, and furthermore derived from breast, colon, lung, prostate and sarcomas. The $IC_{50}$ ranged between 0.09 and 1.14 $\mu$g/ml. Compared to the established "anticancer agents like Cisplatin, Carboplatin or 5-Fluorouracil, Breastin showed a higher overall activity and also a marked tumor selectivity.

2. Effect of Breastin in patient derived tumor models studies in the tumor colony assay invitro

Breastin was studied in 67 human tumor models which were established by implanting patient tumors subcutaneously into nude mice. These tumors remain also in serial passage typical properties of the donor tumors including heterogeneity and tumor sensitivity profiles. Breastin was studied in 6 dose levels from 0,3 ng/ml up-to 30 mg/ml. There was a clear dose response relationship. Overall, Breastin was active in 24/67 tumors (36 %). Activity was seen in cancers of the bladder, CNS, colon, lung, ovary, prostate, renal and uterus carcinomas as well as in melanomas and pleuramesotheliomas. Resistant were cancers of the pancreas and sarcomas.

B. Antiviral Effects and Immunstimulation:

The antiviral and immunomodulator effects of an oral preparation comprising the cold extract according to the present invention (designated as Breastin in the following) were investigated as follows:

The antiviral mechanism of Breastin was studied extensively and it was found that Breastin is one of the interferon (IFN) inducers - a special group of potential antiviral compounds IFN - inducers represent a special group of potential antiviral inducing activity. These inducers have many requirements, among these are:

(a) a high IFN-inducing activity.
(b) good solubility in aqueous biological fluids.
(c) broad therapeutic margin.
(d) wide range of antimicrobial activity.

It appears that IFN-inducers stimulate the IFN production in different immunological effector cells and organs and that may explain their efficacy in hepatitis B and C, infuenza A and B and HIV treatment.

Breastin has significant antiviral activity against HIV-1, influenza viruses A and B and polio virus type 1 where the $IC_{50}$ were found to be 0.1, 0.3 and 0.5 $\mu$g/ml, whereas no antiviral activity was demonstrated by Breastin on Herpes virus simplex type 1, Reo and yellow fever viruses ($IC_{50}$ found to be >20 and > 100 $\mu$g/ml respectively).

Release of cytokines from human PBMCs give an indication which immunological effector cells are being stimulated by Breastin. There has been investigated the effect of Breastin on PBMCs of 3 healthy donors and found a strong release of IL-6, TNF-alpha and IL-1B and some release of Interferon-gamma at dose levels which are not cytotoxic. These cytokines are released mainly from macrophages / monocytes, TNF-alpha and IL-6 from lymphocytes and natural killer cells. Therefore, Breastin has demonstrated immune stimulatory effects.

Breastin has also demonstrated an immune-modulatory effect as shown from the primary humoral immune response to sheep red blood cells and P-815 mastocytoma cell lines ($IC_{50}$ = 0.31 and 2.5 $\mu$g/ml respectively). In addition, it was found that Breastin had no effect on P-815 tumor cells using higher doses and this was another confirmatory evidence concerning the immune modulatory role of Breastin. On the other hand, a significant anti-complimentary activity was demonstrated by Breastin ($IC_{50}$ < 0.1 $\mu$g/ml). Finally, the effect of Breastin on the IFN-production was also studied. All tested concentrations were active in IFN-production *in vitro*.

The water-soluble sterile cold extract according to the present invention exhibits a new property, in particular a IFN-inducing activity. This cold extract can be used in treating viruses, such as HIV-1, Hepatitis B and C and influenza viruses as well.

Several criteria were adopted to review the activity of Breastin including the physico-chemical properties and a consistent biological activity both *in vitro* and *in vivo*.

**1. *In vitro* studies**

1.a Primary humoral immune response to sheep red blood cells

Mouse spleen cells (8-10 weeks old) were co-cultured with sheep erythrocytes for 3 days in 1 ml final volume using 24 well plates. The lymphocytes were harvested, washed and plated (1 x $10^5$ cells) onto softagar with fresh antigen. Complement (guinea pig serum), was added after about 60-90 minutes incubation period and incubation was continued for another 60 minutes. Then, the test was evaluated by using a microscope (plaques counting). After 72 hours of incubation, the lymphocytes were sensitized to the antigen when incubated with antigen again, B-lymphocytes secretes specific antibody which binds to the antigen of the secretary lymphocytes. Addition of complement causes lysis of the antibody coated erythrocytes yielding a plaque and each plaque represents a single antibody producing cell. The results showed that the $IC_{50}$ of the extract was 3.1 ($\mu$g/ml) indicating the immune-modulatory effect.

2.a One-way mixed lymphocyte reaction (MLR)

Spleen cells obtained from female albino mice (8-10 weeks old) were coincubated for 5 days with mitomycin C treated spleen cells from some specific female mice (8-10 weeks). The cells induce a proliferative response in albino C spleen cells which can be measured by labeled precursor incorporation into the DNA. Since the stimulator cells, were mitomycin C treated, they do not respond to the albino C cells with proliferation but they do retain their antigenicity.

3.a Cytotoxic and cytostatic activity using the P-815 mastocytoma cell-line

A 96-well plate was filled with a tissue culture medium (100 $\mu$l/well), the extract was added to the top row in a 25 $\mu$l aliquote, mixed, 25$\mu$l was removed and added to the next lower row and this process was repeated until the end of the plate was reached. The last 25 $\mu$l were discarded. 100 $\mu$l of cell suspension (P-815 mastocytoma cells, about 30000 cells/well) was then added to each well and incubated for 2 days at 37°C. The proliferation of cells was assessed by the use of a cell counter. The plates were centrifugated, the supernatant was discarded and the cells were washed with phosphate-buffer, saline and a 50 $\mu$l/well of Triton-x solution was added followed by shaking the plates for 5-10 minutes. The plates were incubated for 60 minutes at 37°C and the substrate was added and followed by the addition of the buffer and the plates were read at 405 nm. The result showed that the $IC_{50}$ of the extract was 2.5 $\mu$g/$\mu$l showing the immune-modulatory effect against the P-815 mastocytoma cell line.

4.a Anti-complementary activity

The inhibition of complement activity was determined as described by Shahat *et al.* Serial and concentrations of the cold sterile extract according to the present invention were prepared. The assay was performed in a v-well microtiter plate. Rabbit complement (C901 virion/serion immunodiagnostic GmbH) and hemolysing anti-sheep erythrocyte serum (C902 virion/serion immunodiagnostic GmbH) were used. 50 $\mu$l of the complement solution (diluted 1:50) were added to 50 $\mu$l of each sample concentration. After an incubation period at 37°C for 30 minutes, 50 $\mu$l of a suspension of sensitized sheep erythrocytes were added to each well. Hemolysis was observed optically after an incubation at 37°C for 60 minutes. Controls consisted of sensitized sheep erythrocytes incubated in buffer (no hemolysis), with working solution complement (100% hemolysis with 1:2 and 1:3 diluted working solution complement (partial hemolysis). The $IC_{50}$ values were calculated and found to be < 0.1 $\mu$g/$\mu$l showing a very significant anticomplementary activity.

5.a Efficacy against reverse-transcriptase (RT) using phosphonoformate as inhibitors of RT

The inhibitory effect of the cold extract according to the present invention against the reverse-transcriptase enzyme was also studied using three different concentrations (0.1, 1 and 10 $\mu$g/ml) *in vitro* and compared with the phosphonoformate (PFA) as inhibitors of RT. The extract demonstrated a significant inhibitory concentration ($IC_{50}$ 4-6 $\mu$g/ml) when compared with PFA ($IC_{50}$ 6-20 $\mu$g/ml).

6.a Ribosome-inactivating protein (RIP) assay (Stripe and Barbieri)

The extract was also studied for its RIP activity (ribosome-inhibiting protein) and showed a significant RIP (less than 1 $\mu$g/ ml).

7.a Interferon (IFN)

The effect of Breastin on INF production in whole blood cell culture was studied *in vitro* using the method according to Wang et al. Different concentrations of Breastin were used on whole blood obtained from healthy donors. The growth of these cells was assayed after 48 h and the activity was determined using the MTT-assay technique. All measurements were performed in duplicate. All tested concentrations of Breastin were active in stimulating the IFN-production in whole blood *in vitro*.

### 2. *In Vivo* Experiments

2.a P-815 Mastocytoma *in vivo*

Female mice (20-22 g) were inoculated in the first experiment with 2 x $10^6$ i.p and with 1 x $10^5$ P-815 tumor cells. In the second experiment the animals were inoculated i.p with 1 x $10^6$ P-815 tumor cell. The treatment with Breastin commenced post tumor inoculation and the survival time was taken as evaluation criteria.

Results

**Table 3:** Effect of Breastin on P-815 mastocytoma *in vivo* using Sc. Route [exp.1]

| Compound | Dose (mg/kg) | Route | Survival time |
|---|---|---|---|
| Control (H$_2$O) | / | s.c. | 13 days |
| Breastin | 0.1 | s.c. | 14 days |
| Breastin | 1 | s.c. | 14.3 days |
| NB: Tumor cells were administered i.p | | | |

**Table 4:** Effect of Breastin on P-815 mastocytoma *in vivo* using i.m route [exp.2]

| Compound | Dose (mg/kg) | Route | Survival time |
|---|---|---|---|
| Control (H$_2$O) | / | i.m | 13 days |
| Breastin | 0.1 | i.m | 14 days |
| Breastin | 1 | i.m | 14.3 days |
| NB: Tumor cells were administered i.p | | | |

The above preliminary experiments have shown that Breastin had no effect on P-815 tumor cells.

2.b B16-Melanoma *in vivo*

Tumor homogenate of B16-Melanoma was implanted subcutanously to BDF$_1$ male mice. Breastin was injected intraperitoneally for 9 days at two dose levels (0.025 and 0.05 ml per mouse). Breastin showed a significant increase in the life span of the BDF$_1$ male mice. The results obtained according to the followings:

$$ILS = (T-C)/C \times 100$$

ILC = increase in the life span.
T = average survival time of the treated mice.
C = average survival time of the untreated mice (control group).
The T/C for the dose 0.025 ml/kg was found to be 135 %, which means an increase in survival time of 35 % which was significant.

**3. Antiviral activity**

The antiviral efficacy of Breastin was *in vitro* investigated at different concentrations (0.01, 0.1, 1 and 10 $\mu$g/ml) and using different tissue-culture-techniques.

3.a Cell lines

1-Mock-infected MT-4 cell lines were used to support the growth of the HIV-1 virus.
2-Mock-infected Vero and BHK-21 cell lines were used to support the growth of HSV-1, Sb1, Reo and yellow fever viruses.
Compound concentrations ($\mu$g/ml) required to reduce the viability of mock-infected MT-4 cells by 50% were determined by the MTT-method. Compound concentrations ($\mu$g/ml) required to reduce the number of mock-infected Vero and BHK-21 cells by 50% were also determined by using the MTT-method.

3.a Viruses

1) Influenza viruses A/Texas/77; A/Philippines/81 and B/Hong Kong/79.
2) Herpes virus simplex type 1 (HSV-1).
3) Poliovirus type 1 (Sb1).
4) Reovirus (Reo).
5) Yellow fever virus (YFV).
6) Human immunodeficiney virus (HIV-1).

Results

The *in vitro* antiviral activity of the cold extract of *Nerium oleander* is shown in Tables 5 and 6.

**Table 5:**

| Compound | $IC_{50}$ (MT-4) | $EC_{50}$ (HIV-1) |
|---|---|---|
| Breastin | 1.0 | 0.1 |
| AZT | > 10 | 0.01 |

Compound concentrations ($\mu$g/ml) required to achieve 50% protection of MT-4 cells from the HIV-1 induced cytopathogenecity, as determined by the MTT-method.

**Table 6:**

| Compound | $IC_{50}$ | | $EC_{50}$ | | | |
|---|---|---|---|---|---|---|
| | [1]Vero | [1,2]BHK2 1 | HSV[3]-1 | Sb[3]1 | Reo[4] | YFV[5] |
| Breastin | 2 | >100 | >2 | 0.5 | - >100 | >100 |

Compound concentrations ($\mu$g/ml) required to reduce the number of mock-infected Vero and BHK21 cells by 50%.

Compound[2] concentrations ($\mu$g/ml) required to reduce the viability of mock-infected BHK-21 monolayers by 50% as determined by the MTT-method.

Compound[3] concentrations ($\mu$g/ml) required to reduce the HSV-1/Sb1 plaque number by 50% Vero monolayer.

Compound[4] concentrations ($\mu$g/ml) required to achieve 50% protection of BHK-21 cells from the Reo virus induced pathogenecity as determined by the MTT method.

Compound[5] concentrations ($\mu$g/ml) required to reduce the YFV plaque number by 50% in BHK-21 monolayer.

Breastin inhibited both strains of influenza viruses replication by log10 ($IC_{50}$ 0.3 $\mu$g/ml).

**Toxicity Studies**

1.B. The Acute Toxicity in mice

The $LD_{50}$ of Breastin in albino Balb/C mice using the intramuscular route (IM) was found to be 0.35 ml/25g (14 ml/kg). The $LD_{50}$ of Breastin using the intraperitoneal (IP) and the subcutaneous (Sc) routes was found to be 0.3 and 0.33 ml/25g respectively. Some apparent side effects like tachycardia, myorelaxation and motoric incoordination were observed with high does. When Breastin was administered in single or repeated doses using the IM, IP and Sc routes and up to 0.15 ml / 25g (6 ml/kg) in normal mice, no side effects were apparent.

2.B. The Subacute Toxicity in mice

The potential lethal effect of Breastin was investigated subacutely over a period of 8 weeks with an experimental design of 3 groups with 50 animals/group. Groups comprised male and female balb C / mice. Group 1 received 0.025 ml daily IP, group II received 0.05 ml daily IP, whereas group III received distilled water daily IP and served as control. Morbidity and mortality checks were made daily. Body measurements were scheduled at day (O) before injection and then on a weekly basis. A number of animals were sacrificed/week. Complete blood counts (CBC) and liver and kidney profiles were done on each sacrifice. In addition, biopsies were taken from the liver and kidney during each sacrifice for histopathology study.

The following observations were recorded during the entire period of the experiment:

1) Animals generally gained weight (all groups) indicating that Breastin is not anorexic at the doses used.
2) Breastin showed no significant changes in the hemoglobin concentration or in the erythrocytes count in all groups at the doses used.
3) No significant changes were observed in the blood indices (MCV, MCH and HCHC).
4) Breastin lead to an increase in the leukocytes count in the experimental groups. Significant increases in the lymphocyte count were recorded at both dose levels.
5) Breastin caused no liver and renal function abnormalities.
6) Four mortalities were recorded in group II during the last three weeks of treatment.
7) Pathologically, three biopsies from kidney taken from group II at week 8 showed some edematous interstitial tissues. Further, three biopsies taken from the liver of group II at week 8 showed multiple focal inflammatory cells in filtered mainly around the central vein with histiocytes. However, some of these changes were also observed in the kidneys and liver biopsies obtained from control untreated animals.

3.B. Special Toxicity Tests for local Tolerance

The effect of Breastin on the eyes of rabbits and on the skin of guinea pigs was studied using standard protocols. Again two dose levels were used in both tests (0.05 and 0.1 ml).

The results of both tests showed that Breastin caused neither erythema nor any cutaneous sensitization in the skin of guinea pigs nor any inflammatory changes in the eyes of the tested rabbits as compared to the untreated controls.

## Chronic Toxicity Studies IM for 6 months in rats

A total of 240 albino rats (120 males and 120 females) were treated for 6 months. The males were approximately 80 days old. The animals were provided with food and *water ad libitum.* Animals were housed in suspended cages (6/cage) and the temperature was maintained at 18-22°C. Animals were divided into three groups (80 animals/group including the untreated control animals).

Group 1: Consisting of 80 rats (both sexes) and received the first dose (approximately 1/10 of the $LD_{50}$ as determined in mice = 0.03 ml).

Group 2: Consisting of 80 rats (both sexes) and received the second dose (approximately half of the $LD_{50}$ as determined in mice = 0.16 ml).

Group 3: Consisting of 80 rats (both sexes) which received distilled water (untreated/control).

Animals were dosed by intramuscular injections. Morbidity and mortality checks were made once daily. Additional observation on the general health conditions were also made on the day of administration. Body weight measurements were scheduled to take place prior to the treatment with Breastin followed by additional body weight measurements on the day of sacrifice (once a month). Daily observations were made throughout the entire period of the experiment (6 months) for all groups to see if any behavioral changes occur due to the administration of Breastin.

Samples of blood were obtained by heart puncture technique and the following tests were performed prior to the treatment with the extract and then followed on the day of sacrifice (once a month). These are:

a) Total red blood cells count (RBC).
b) Hemoglobin concentration (HGB).
c) Total white blood cell count (WBC).
d) Differential white blood cell count.
e) Thrombocyte count.

Liver parameters:

f) Aspartate transaminase (AST).
g) Alanine transaminase (ALT).
h) Alkaline phosphates (ALP).
i) Total albumin (TA).

Kidney parameters:

j) Urea.
k) Creatinine (Crea).

Results and Discussion

1. All animals remained healthy up to the time of sacrifice. No adverse reactions were noted at the time of observation during the entire period of the experiment. Six mortalities were recorded in group 2, 2 mortalities were recorded in group 1 and only one mortality was recorded in the untreated controls.

2. The gross-anatomy of the organs of the sacrificed animals in all groups was inspected and biopsies were excised from the kidneys and the livers, prior to the treatment with Breastin and then followed on the day of sacrifice (once a month) to look for any gross-abnormalities or histopathological change The gross-anatomy of the organs of sacrificed animals looked morphologically normal and no gross-abnormalities were detected.

3. All results were statistically analyzed using different methods of analysis and they indicated:

a) The statistical analysis performed on the blood cell count throughout the entire period of the experiment (6 months) showed that there were no significant differences between all the groups.
b) The statistical analysis performed on the biochemical results indicated no significant differences in the kidney function tests between the treated groups I and II when compared with the control groups.
c) Some significant differences were detected in some of the liver function tests between two treated groups I and II. Group II which received the high dose showed significant increase in both the AST and ALP enzymes compared to group I, whereas no significant differences were detected in the ALT enzyme in both the treated groups.
d) The histopathological studies performed on biopsies obtained from the kidneys and the livers gave also normal results. However, the microscopic anatomy performed on biopsies taken from the liver of the dead animals showed some changes such as infiltration of inflammatory cells and passive congestion, but no central necrosis was detected.

**Clinical Studies / Phase I/II-Studies**

A. Anticancer Activity of Breastin

**Phase I study**

In a phase I study Breastin was tested in 35 patients, 25 presented advanced breast cancers and 10 with colon cancer. Breastin was given IM and escalated to daily 3 to 4 mg (0.4 to 0.6 ml) for 4 months followed by oral administration with 10 to 11 mg (35 to 40 drops per day) which were later adjusted to 15 to 20 drops per day. The limiting toxicity was risen in body temperature to 38 to 38.2 °C; vomiting, nausea, diarrhea and fatigue was observed in 30 % of the patients. There were no treatment related death. Tumor regressions and clinical improvements were seen.

**Phase II studies in 383 patients with solid cancers and hematologic malignancies**

Table 7 summarizes the diagnoses, the number of patients included and the response rate. Patients presented the following diagnoses: Breast cancer 150, colorectal 57, prostate, 32 and lung 33. In addition 14 patients were treated with stomach, 15 with basal carcinoma of the skin, and less than 10 with melanomas, glioblastomas, osteosarcomas, thyroid, nasopharynx and bladder cancers were included. The response rate was defined by complete and partial responses as well as tumor stabilizations which were seen between 40 and 70 %. Part of the patients were pretreated with chemotherapy. Overall, this response rate compares very favourable with other registered agents in the respective tumor entity.

In addition, Breastin was also studied in hematologic malignancies like 13 chronic lymphocytic leukemias (CLL), 10 acute lymphoblastic leukemias, Hodgkin's and non-Hodgkin's lymphomas, and malt lymphoma. The response rate was highest in Hodgkin's lymphoma (43 %), in the other diseases up to 20 %.

**Table 7:** Summarized anticancer activity of Breastin in phase II studies and late phase II during 1988 up to date.

**- Solid cancers and hematologic malignancies -**

| Types of Cancer | No. of patients | % Response* |
| --- | --- | --- |
| Breast | 150 | 70 |
| Colorectal | 57 | 54 |
| Prostate | 32 | 69 |
| Lung | 33 | 64 |
| Stomach | 14 | 57 |
| Skin basal cell | 15 | 67 |
| Osteosarcoma | 13 | 46 |
| Skin / Melanoma | 4 | 50 |
| Glioblastoma multiforma | 5 | 40 |
| Nasopharynx | 6 | 50 |
| Thyroid | 8 | 63 |
| Larynx / sqamous cell | 3 | 72 |
| Bladder and ureter transitional | 5 | 53 |
| Hodgkin's lymphoma | 7 | 43 |
| Non-hodgkin's lymphoma | 5 | 20 |
| Malt lymphoma | 3 | 33 |
| Chronic lymphocytic leukemia (CLL) | 13 | 8 |
| Acute lymphocytic leukemia (ALL) | 10 | 20 |

\* response, complete and partial responses plus stabilisations

Case Report 1: Breast cancer with secondary brain metastasis

A terminal case of breast cancer with a history of right mastectomy. The left breast on examination was free and the left axilla showed painful, hard, multiple mobile lymph nodes (3x4 cm) with no skin involvement. According to a CT-scan of the brain, a small (< 1cm) enhancing lesion was seen in the right parietal region in the midline and no evidence of surrounding edema was noted. The lesion was considered to be a metastasis. When the patient presented to the clinic, she was very sick with a huge edema in the right axilla and a loss of vision. She started the treatment with Breastin using both routes (IM and PO). Three weeks later the patient showed significant changes in the reduction of the right axillary edema and the severity of pains decreased. Five weeks after the treatment with Breastin the patient gained weight (3kg) and the size of the left axillary lymph nodes showed significant reduction in size. The treatment with Breastin was continued for almost one year and during said period the patient was doing well both physically and clinically. A CT-scan performed after one year of treatment with Breastin showed that the size of the lesion in the parietal region and the huge edema in the right axilla was reduced significantly. Unfortunately, the patient died after surgical operation.

Case Report 2: A Recto-Sigmoid Cancer stage Duke D

A known case of a recto-sigmoid cancer stage- Dukes D was presented with colicky pains in the lower abdomen and with severe pains at the anal area, etc. She started the treatment with Breastin using both the IM and PO routes. Two months later a CT-scan was performed and showed that the internal iliac lymph node was getting smaller compared to the previous scan before treatment with Breastin. The CEA at the same time decreased significantly to 4 nanogram (it was much higher before Breastin treatment). Colonoscopy was performed after 4 months of treatment with Breastin and showed that the tumor size inside the lumen was of egg size which was smaller than before. Furthermore, the mass became very friable and a regression of the size was noticed. All these findings were accompanied by improvement of the appetite and the disappearance of pains in the abdomen and the perianal area and no more constipation associated with bleeding. The treatment with Breastin continued for about two years during which a careful follow-up was performed using CT-scan, laboratory tests etc. Indeed the patient looked clinical better and significant improvements were achieved and the tumor mass decreased significantly. In addition, the physical performance of the patient was almost normal and almost all the laboratory findings were within the normal limits. A CT-scan was performed after two years of treatment with Breastin which showed a complete regression of the mass from the lumen. The patient continued the maintenance treatment for another two years later.

Case Report 3: Osteogenic Sarcoma

A terminal case of osteogenic sarcoma which was diagnosed by a biopsy from the proximal left tibia. His family refused to give him either chemotherapy or radiotherapy and they insisted that he should be treated with Breastin. He started the treatment with Breastin using both the IM and PO routes. One month later he showed a bleeding in the tibial region. A bone scan revealed that there was a new periosteal growth and new osteocytes growing in the upper left tibia. The compression at the surroundings was also reduced and the swelling was reduced too. A chest X-ray done at the same time showed no lung metastasis. Six months later another CT-scan was performed of the upper tibial region which revealed a healing process and the swelling in the area was significantly reduced. One year after treatment with Breastin another CT-scan was performed and more osteocytes were observed and more periosteal growth was note. A chest X-ray was done at the same time and showed no lung metastasis. 14 months later another CT-scan was performed which revealed complete healing and remission to the upper left tibia. Another chest X-ray was performed at the same time and indicated that the lungs are free from any metastasis. The patient went on for maintenance therapy for another 14 months with Breastin. The patient was still ok after almost 4 years of treatment with Breastin, but died later when he used other treatments of unknown source.

Case Report 4: Hodgkin's Lymphoma

A 55-years old lady presented with a pain in the left chest, cough and weakness. The clinical examination revealed loss of breathing sound in the left lung. A chest X-ray confirmed the presence of fluid in the same lung and a tumor mass. A fine needle aspiration was performed and was diagnosed as malignant lymphoma. The patient refused to take chemotherapy and decided to take Breastin instead. One month after the treatment with Breastin, an X-ray report showed a significant regression of the tumor mass. Three months later another X-ray was performed and showed that the tumor mass has regressed to about half and the physical performance improved remarkable too. Six months later another X-ray scan was performed and showed the complete regression of the mass. However, she had been advised to continue the treatment with Breastin. So she started the maintenance treatment and continued the treatment for another six months. She had indeed no more complains and she was physically and psychologically ok. Another X-ray was done after one year and again no mass was demonstrated. A biopsy was taken for pathological study and no pathological findings were noted.

Case Report 5: Glioblastoma multiforma

A 49 years old right-handed man, presented with a right homonomous hemianopia. A CT-scan of the head revealed a mass lesion in the left parietal-occipital region. There was a significant surrounding edema. The patient reported visual changes occurring over a several week interval prior to his evaluation at the hospital. His wife noted increasing difficulty with confusion. The patient revealed on examination a dense right homonomous hemianopia as well as evidence of a parietal-lobe syndrome. The patient was admitted to the hospital to undergo craniotomy with tumor debulking of the brain tumor and biopsy. The results of the fresh frozen section obtained at the time of craniotomy showed glioblastoma multiforma belonging tao high grade astrocytoma. In addition to craniotomy and radiation therapy, the patient also was treated with chemotherapy. However, the condition of the patient deteriorated and the hospital discharged him. He and his wife decided to take Breastin as the last choice. He started the treatment with Breastin. However, he was in a very poor medical condition. He was being carried on a stretcher and unable to stand up due to the feebleness occurring in his legs and arms. Two months after treatment with Breastin using both routes, the patient was doing well. The feebleness in his legs and arms as well as his uncomfortable and agitated appearance had almost disappeared. Five months after the treatment with Breastin the patient could walk without any help and a brain CT-scan demonstrated considerable regression of the mass. Another brain CT-scan performed after 10 months revealed "no relapse of the tumor lesion" and the patient was feeling well. Another brain CT-scan performed after 18 months revealed no change with respect to the previous CT-scan. The patient was advised to start a maintenance therapy for another 18 months. Later on, the patient lived normally and he was free from any symptoms.

B. Anti-HIV Activity of Breastin

A total of 14 male and female patients with confirmed HIV/AIDS patients, aged 12-45 years (3 drug users, 6 heterosexual, 4 homosexual and 1 received contaminated blood during surgery) were treated with Breastin, during the period between 1993-2003. These patients can be classified as follows:

3 with class A HIV disease; 6 with class B HIV disease and 5 with a $CD^+4$ lymphocyte count $< 200\mu$l.

Ten of these cases received prior treatments (either Retrovir or AZT; antibiotics and some other antiviral agents) in some medical centers before they decided to receive Breastin. The other four patients received no previous treatment when they were referred to the center. Most of the patients were suffering from the following symptoms:

(a) Continuous weakness and insomnia.
(b) Severe body weight loss and loss of appetite.
(c) Severe diarrhoea.
(d) Presence of opportunistic infections such as pneumonia, *Herpes zoster,* moniliasis, oral and esophageal candidiasis, etc.
(e) Herpes infection especially at the penis of the males.
(f) Hepatomegally.
(g) Loss of energy.
(h) Depression.

Three routes of administration were followed:

(a) Intramuscular route (IM).
(b) Oral route (PO).
(c) Topical route.

For the intramuscular route, a single daily injection of about 2-4 mg of the active ingredients of Breastin was administered daily for 6 days/week (0.2 - 0.4 ml). The oral route was used as adjuvant to the IM-route. The oral administration was 10-11 mg in 15 drops/three times daily after meals. The topical route was used only to those patients presenting vascular lesions on the limbs e.g. Kaposi's sarcoma.

Case Report 1

A 12 years old girl was presented in a very poor medical condition:

(a) Suffering from chronic diarrhoea and cough.
(b) Serious loss of the hypodermis adipose.
(c) Severe body weight loss (15 Kg).
(d) Hepatospleenomegally (13 cm x 7 cm).
(e) Chest pain during respiration at both mammary region.
(f) Severe fatigue.

The girl has received blood transfusion for several times and the laboratory finding showed that the HIV Antigen and HIV-Antibody tests were positive. The liver enzymes (ALT, AST and the GGT) were above normal values (410, 190 and 420 u/L respectively). She was anemic and the PCV value was less than 26% the total RBC 1500/$\mu$l and the WBC was less than 800/$\mu$l. The stool examination revealed an infection with *Salmonella typhimurium.* The girl was first treated with some antibiotic and antifungal drugs for about 2-3 weeks. This is followed later by Breastin treatment using both the IM and the oral routes. Four weeks after the treatment with Breastin she recovered from the oral infection and from pneumonia and no more loss of weight could be observed. The frequency of diarrhoea also decreased significantly and both the livers and the spleen showed a decrease in size. The laboratory findings indicated a decrease in the ALT, AST and GGT-enzyme activities. Following the result obtained it was decided to continue the treatment with Breastin for another period of time. The treatment continued for another 3 months and the following was observed

(a) Significant improvements in the general condition of the girl was observed.
(b) Most of the signs noticed before treatment disappeared completely (including fatigue, diarrhoea, body weight loss, hepatospleenomegally, etc).

(c) No side effects were noticed.

(d) The viral RNA load was reduced to 50% when compared before treatment.

(e) The Elisa and Western Blott tests showed the presence of the virus.

Therefore, it was decided to continue the treatment for another period of time. The patient continued the treatment for another 5 months using the same protocol. The patient's general health condition became very good and most of the undesirable symptoms disappeared. The laboratory analysis performed on the blood and the patients indicated the followings:

(a) The viral RNA load was reduced to more than 78% when compared before administering the treatment.

(b) Different blood parameters were normal.

(c) The ALT, AST and GGT-enzyme activities readings were normal.

(d) The Elisa and Western Blott tests showed the presence the virus.

These results were very promising, however, it was very difficult to interpret the results, that is, the presence of the virus. It can be assumed at this stage that Breastin had a significant effect on the immune system. In other words, Breastin stimulated the immune system of the patient which resulted in the recovery of the patient. Therefore, it was decided to look at other parameters such as Immunoglobulins IgG, IgA and IgM etc. The patient continued the maintenance therapy for about 9 months. Follow-up studies were performed after the maintenance therapy and the patient was ok clinically and all the laboratory analysis performed were normal.

<u>Case Report 2</u>

A 35 years old homosexual man was diagnosed as HIV-1 positive patient. Immediately after diagnosis he started the AZT regime treatment. This patient was suffering from severe diarrhoea with bloody discharge, cough, severe weight loss, fatigue and he also developed Herpes zoster lesion. However, due to the severe side effects developed by the AZT he discontinued the treatment after 8 weeks. He referred to the center with complicated side effects such as no appetite with dysphagia mainly for hard food, infection with candida in the eosphagus, severe chest pain especially during swallowing, severe diarrhoea, upper abdominal pain, hair loss, fatigue, no libido and he was in a deep depression. Before he started the treatment with Breastin, the following tests were formed:

(a) CBC: most of the parameters were normal except that the total WBC was low (1950).

(b) Liver parameters were elevated, especially the ALT, AST and the GGT:

| | |
|---|---|
| ALT | 360 u/l. |
| AST | 140 u/l. |
| GGT | 401 u/l. |

(c) Immunoglobulins:

| | |
|---|---|
| IgG | 2810 mg/dl. |
| IgA | 594 mg/dl. |
| IgM | 930 mg/dl. |

(d) HIV 1 and 2 RNA detection by polymerase chain reaction (PCR) showed that the viral RNA concentration was very high and the patient was very happy with the treatment. The psychological performance changed and he gained weight and hair growth restored. The patient started to take Breastin using both routes (IM and the oral routes). Six weeks later the response of the patient to Breastin was remarkable and this was obvious on the general health condition of the patient. Weakness, fatigue, anorexia decreased gradually. Dysphasia, candidal esophageal infection and chest pain disappeared. No blood test performed during this period and the treatment continued for another 12 weeks. The patient was doing well and most the clinical symptoms disappeared.

A blood test was performed after 5 months of treatment with Breastin and revealed:

(a) All blood parameters were normal including the total WBC count.

(b) The liver enzymes level were almost normal.

(c) The immunoglobulins values back to normal level.

(d) The HIV 1- and 2 RNA value decreased significantly to almost 70%.

It is important to point out that most patients treated with the extract according to the present invention showed dramatic improvements in the general health and enjoyed sleeping without night sweets and gained weight.

To conclude, it can be noted that Breastin has the following advantages when used to treat HIV/AIDS patients:

(a) Within two months of treatment with Breastin, the total WBC and platelets counts returned to normal condition.
(b) Fungal infection in the mouth due to candida and diarrhoea due to *Salmonella* also disappeared within 2-3 months after the treatment.
(c) The loss of body weight and fatigue disappeared within 3-4 months after the treatment.
(d) Within about 6-7 months treatment with the extract, herpes infection also disappeared completely.
(e) Within 2-4 months treatment the enzymes ALT, AST and ALP decreased to its normal range.
(f) Chronic abscess disappeared within 2 months.
(g) The patients showed good psychological performance after 2-3 months of treatment with the extract.

## C. Anti-HCV Activity of Breastin

During the period between 1996-2000, a total of 10 patients (6 adult males and 4 adult females) aged 39 - 51 years old were treated with Breastin. Six of these patients received previously interferon (3 million units per week for a period of 6 months). However, these patients showed relapse at the end of the treatment with interferon. The remaining four patients received no other treatments. The following criteria were followed to enroll patients in this novel preparation:

(a) Confirmed laboratory diagnosis (marked elevation of amino transference enzymes up to 10 folds).
(b) Positive liver biopsy.
(c) Confirmed anti-HCV in the serum.
(d) Clinical symptoms (fever, fatigue, hepatomegally, epistaxis, anorexia etc.).

Case Report 1

A 51 years old lady has been diagnosed as HCV-positive since 1995. She was treated previously with interferon for 6 months, however, she showed a relapse at the end of the treatment. The blood tests performed two months after the treatment with interferon are shown in the table hereinbelow.

Physical finding

The patient when presented to the clinic was anemic, anorexic, pale, jaundiced, fatigued and she was unable to speak. She started taking Breastin on March 1996 using the oral route. The initial dose was 10 drops/three times daily after meals. Within four weeks after the treatment, the patient showed rapid improvements in the clinical symptoms such as the disappearance of fever and fatigue and the appetite improved significantly. A blood test performed after 4 weeks treatment with Breastin showed improvement of most liver parameters:

**Laboratory Findings of a Patient with HCV, treated with Breastin**

| Parameter | Before Breastin | After 1 month | After 3.5 months | After 6 months | After 10 months |
|---|---|---|---|---|---|
| ALT | 250 u/l | 160 | 105 | 41 | 35 |
| AST | 250 u/l | 145 | 95 | 35 | 22 |
| Albumin | 59g/dl | 40 | 29 | 42 | 82 |
| Total bilirubin | 6 mg/dl | 3.3 | 1.6 | <1 | <1 |
| Direct bilirubin | 3.9 mg/dl | 2.3 | 1.2 | <1 | <1 |
| Alkaline phosphatase | 312 u/l | 270 | 211 | 149 | 140 |
| **HCV-RNA (PCR)** | **750,000 u/ml** | **750,000** | **410,000** | **170,000** | **NEGATIVE** |

The patient improved significantly and she was impressed by the laboratory results. The dose of Breastin was adjusted to be 15 drops/three times daily. In mid July the patient came to the clinic for a medical check up. She looked almost healthy and she was energetic and most of the clinical symptoms disappeared. A blood test performed after almost three and a half months treatment showed further improvement.

Physical findings showed almost normal behavior and improved significantly compared to the previous period. In mid September she visited the clinic for another medical check up and after almost six months of treatment with Breastin. Indeed she was almost normal physically and clinically.

In January 1997 she had another visit to the clinic for another medical check up and after almost 10 months treatment with Breastin. Physically and clinically the patient was normal. The blood test performed showed normalization of all liver parameters and HCV-RNA determination by PCR was now negative.

A complete disappearance of the anti-HCV after 10 months treatment with Breastin was unexpectedly observed, which demonstrates the activity of Breastin against HCV.

During the period between 2001 to 2005 and due to the significant and encouraging results obtained during the period between 1996 - 2000, additional clinical studies were performed with Breastin and more than 45 patients (males and females) aged 15 - 65 years old were enrolled. 40 of these patients were previously treated with different antiviral drugs such as interferon, Amantidine and Ribivirin. However, these patients showed relapse at the end of the treatments, five of the patients received no prior treatments. Again the above criteria were applied to enroll these patients in this herbal treatment. Indeed, almost more than 95% of these patients showed physical and clinical improvements following Breastin treatment. Only seven patients were withdrawn from the treatment.

The following conclusions regarding the HCV-patients must be drawn:

(a) Within less than two months of treatment with Breastin most of the liver enzymes improved significantly.
(b) The total and direct bilirubin also improved significantly in most of the patients during the first six weeks of treatment with Breastin.
(c) Disappearance of the abdominal pains and improvement of appetite during the first 6-8 weeks of treatment with Breastin.
(d) Patients were more energetic and they were psychologically improved.
(e) According to the laboratory findings it was noticed that the HCV-RNA (PCR) count declined 80000/ month on an average.
(f) The most interesting result obtained on HCV-patients was the disappearances of the anti-HCV from some of the patients after 9-10 months treatment with Breastin which was really a big surprise. Further studies are in progress to elucidate the mechanism of Breastin on HCV-virus and more patients are now enrolled in the treatment.

### D. Anti-HBV Activity of Breastin

During the period between 1998 - 2004, a total of 10 patients, aged 21 - 54 years old were put on Breastin treatment. Six of these cases were classified as acute and four were classified as chronic HBV. The following criteria were followed to enroll patients in this treatment:

(a) Confirmed anti-HBSAg in the serum.
(b) Marked elevations of the aminotransferase enzymes.
(c) Positive liver biopsy.
(d) Clinical findings such as hepatomegally, ascites, fatigue, anorexia, etc.

### Case Report

A 50 years old gentleman was diagnosed in the hospital with acute clinical hepatitis B. The blood test showed:

HBSAg = 8.2 (positive)
HBCABIGM = positive
HbeAb = negative

He started the oral treatment with Breastin on the next day. Initially he took 7-8 mg/daily and after two weeks the dose was increased up to 10-11 mg/daily (10 drops/three times daily). He showed no side effects. The treatment continued for three months. The blood test performed immediately after 3 months showed the following:

HbsAg = negative
HbsAb = negative

Conclusion

Breastin is a herbal non-toxic aqueous solution that contains both polar and nonpolar compounds that can stimulate and/or modulate the immune system of the body through enhancing the production of pro-inflammatory cytokines such as IL-2, IFN- and TNF-α; IL-6 and IL-1B that are able to stimulate the T-cytotoxic lymphocytes, B-lymphocytes, natural killer cells and macrophages to kill virally infected cells either in specific type of recognizing tumor antigens by T-cytotoxic cells or less specific by NK-cells. In addition, Breastin showed no long-term toxicity when studied in animals. Further, it has no mutagenic effects at very high concentration up to 50 μg/ml as indicated when studied on two strains of *Salmonella typhimurium* TA 1530, TA 1537.

Breastin showed clinical efficacy against Hepatitic B and C, HIV-1, influenza viruses A and B strains.

**Claims**

1. Use of a sterile water-soluble non-toxic pyrogen-free cold extract, obtainable by a method of preparing a cold extract from *Nerium oleander* containing oleandrin and other digoxin-type glycosides comprising the steps of:

   (i) soaking a dried powder, obtained from the leaves of *Nerium oleander,* in a sterile medium selected from distilled water, a water/ethanol mixture, a water/methanol mixture, methanol or ethanol, for a period of 1 to 50 hours at a temperature in a range of from 0 to 30°C,
   (ii) filtering the resultant solution under sterile conditions,
   (iii) optionally adjusting the volume of the resultant solution to a predetermined volume, followed by:
   (iv) filtration under sterile conditions, and
   (v) optionally spray drying or freeze drying of the filtrate under sterile conditions,

   in the preparation of a medicament as a supplementary medication in cancer therapy in combination with taxol, adriamycin, cisplatin, 5-fluoro-uracil, alimta, cyclophosphamide, mitomycin-C, navelbine, taxotere or topotecan by enhancing the antitumor acitivity and reducing side effects and stimulating immunological effector cells.

2. The use according to claim 1, wherein the active ingredients of *Nerium oleander* are extracted from the leaves of *Nerium oleander* having a length of 16 to 19 cm which are harvested during a period of from August to December.

3. The use according to anyone of claims 1 and 2, wherein step (i) is conducted at room temperature and under sterile conditions.

4. The use according to anyone of claims 1 to 3, wherein in step (iv) a filter having a pore size in a range of 0.22 to 0.45 μm is used.

5. The use according to anyone of claims 1 to 4, wherein the sterile extract has endotoxin concentrations of less than 30 units/ml.

6. The use according to anyone of claims 1 to 5, wherein the extract is obtainable by:

   (a) rinsing the ground powder of *Nerium oleander* in a sterile medium selected from distilled water, a water/ethanol mixture, a water/methanol mixture, methanol or ethanol, for 4 to 24 hours at a temperature in a range of 0 to 30°C, and
   (b) filtering the obtained suspension several times and adjusting the pH value to a range of 5.7 to 6.0 under laminar flow conditions using a filter having a pore size in a range of 0.22 to 0.45 μm.

7. The use according to anyone of claims 1 to 6, wherein the sterile water-soluble extract is to be used by intramuscular, oral, rectal and intravenous administration.

**Patentansprüche**

1. Verwendung eines sterilen, wasserlöslichen, nicht-toxischen, Pyrogen-freien kalten Extrakts, erhältlich durch ein Verfahren der Herstellung eines kalten Extrakts aus *Nerium oleander,* enthaltend Oleandrin und andere Digoxin-artige Glycoside, umfassend die Schritte:

(i) Einweichen eines trockenen Pulvers, das von den Blättern von *Nerium oleander* erhalten wurde, in einem sterilen Medium, ausgewählt aus destilliertem Wasser, einem Wasser/Ethanol-Gemisch, einem Wasser/Ethanol-Gemisch, Methanol oder Ethanol, für eine Dauer von 1 bis 50 Stunden bei einer Temperatur in einem Bereich von 0 bis 30°C,

(ii) Filtern der sich ergebenden Lösung unter sterilen Bedingungen,

(iii) gegebenenfalls Einstellen des Volumens der sich ergebenden Lösung auf ein vorbestimmtes Volumen, gefolgt von:

(iv) Filtern unter sterilen Bedingungen, und

(v) gegebenenfalls Sprühtrocknen oder Gefriertrocknen des Filtrats unter sterilen Bedingungen,

in der Herstellung eines Medikaments als ergänzende medikamentöse Behandlung in der Krebstherapie in Kombination mit Taxol, Adriamycin, Cisplatin, 5-Fluorouracil, Alimta, Cyclophosphamid, Mitomycin-C, Navelbine, Taxotere oder Topotecan durch Verbessern der antitumoralen Aktivität und Reduzieren von Nebenwirkungen und Stimulieren immunologischer Effektorzellen.

**2.** Verwendung nach Anspruch 1, wobei die aktiven Bestandteile von *Nerium oleander* aus den Blättern von *Nerium oleander,* die eine Länge von 16 bis 19 cm aufweisen, die während eines Zeitraums von August bis Dezember geerntet werden, extrahiert werden.

**3.** Verwendung nach einem der Ansprüche 1 und 2, wobei Schritt (i) bei Raumtemperatur und unter sterilen Bedingungen durchgeführt wird.

**4.** Verwendung nach einem der Ansprüche 1 bis 3, wobei in Schritt (iv) ein Filter, der eine Porengröße im Bereich von 0,22 bis 0,45 μm aufweist, verwendet wird.

**5.** Verwendung nach einem der Ansprüche 1 bis 4, wobei der sterile Extrakt Endotoxin-Konzentrationen von weniger als 30 units/ml aufweist.

**6.** Verwendung nach einem der Ansprüche 1 bis 5, wobei der Extrakt erhältlich ist durch:

(a) Spülen des gemahlenen Pulvers von *Nerium oleander* in einem sterilen Medium, ausgewählt aus destilliertem Wasser, einem Wasser/Ethanol-Gemisch, einem Wasser/Methanol-Gemisch, Methanol oder Ethanol, für 4 bis 24 Stunden bei einer Temperatur im Bereich von 0 bis 30°C, und

(b) mehrmaliges Filtern der erhaltenen Suspension und Einstellen des pH-Werts auf einen Bereich von 5,7 bis 6,0 unter Laminarströmungsbedingungen unter Verwendung eines Filters, der eine Porengröße im Bereich von 0,22 bis 0,45 μm aufweist.

**7.** Verwendung nach einem der Ansprüche 1 bis 6, wobei der sterile, wasserlösliche Extrakt durch intramuskuläre, orale, rektale und intravenöse Verabreichung zu verwenden ist.

**Revendications**

**1.** Utilisation d'un extrait froid sans pyrogène non toxique soluble dans l'eau, pouvant être obtenu par un procédé de préparation d'un extrait froid de *Nerium oleander* contenant de l'oléandrine et d'autres glycosides de type digoxine comprenant les étapes consistant à :

(i) immerger une poudre séchée, obtenue des feuilles de *Nerium oleander,* dans un milieu stérile choisi parmi l'eau distillée, un mélange eau/éthanol, un mélange eau/méthanol, du méthanol ou de l'éthanol, pendant une période de 1 à 50 heures à une température comprise entre 0 et 30°C ;

(ii) filtrer la solution obtenue dans des conditions stériles,

(iii) ajuster éventuellement le volume de la solution obtenue à un volume prédéterminé, puis

(iv) filtrer dans des conditions stériles, et

(v) éventuellement sécher par pulvérisation ou lyophiliser le filtrat dans des conditions stériles,

dans la préparation d'un médicament utilisé comme médicament supplémentaire dans le traitement du cancer, en combinaison avec le taxol, l'adriamycine, la cisplatine, le 5-fluoro-uracil, l'alimta, le cyclophosphamide, la mitomycine-C, la navelbine, le taxotère, ou le topotécane en augmentant l'activité anti-tumorale et en réduisant les effets secondaires et en stimulant les cellules effectrices immunologiques.

**2.** Utilisation selon la revendication 1, dans laquelle les ingrédients actifs du *Nerium olander* sont extraits des feuilles du *Nerium olander* ayant une longueur de 16 à 19 cm, qui sont récoltées pendant une période s'étalant entre août et décembre.

**3.** Utilisation selon l'une quelconque des revendications 1 et 2, dans laquelle l'étape (i) est réalisée à la température ambiante et dans des conditions stériles.

**4.** Utilisation selon l'une quelconque des revendications 1 à 3, dans laquelle dans l'étape (iv), un filtre ayant une taille de pore comprise entre 0,22 et 0,45 $\mu$m est utilisé.

**5.** Utilisation selon l'une quelconque des revendications 1 à 4, dans laquelle l'extrait stérile a des concentrations d'endotoxine inférieures à 30 unités/ml.

**6.** Utilisation selon les revendications 1 à 5, dans laquelle l'extrait peut être obtenu en :

(a) rinçant la poudre broyée de *Nerium olander* dans un milieu stérile choisi parmi l'eau distillée, un mélange eau/éthanol, un mélange eau/méthanol, du méthanol ou de l'éthanol, pendant 4 à 24 heures à une température comprise entre 0 et 30°C, et
(b) filtrant la suspension obtenue plusieurs fois et en ajustant la valeur de pH à une gamme de 5,7 à 6,0 dans des conditions de flux laminaire, en utilisant un filtre ayant une taille de pores comprise entre 0,22 et 0,45 $\mu$m.

**7.** Utilisation selon l'une quelconque des revendications 1 à 6, dans laquelle l'extrait soluble dans l'eau stérile doit être utilisé pour une administration par voie intramusculaire, orale, rectale et intraveineuse.

**EP 1 976 542 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- NZ 514794
- WO 0016793 A
- EP 0398313 A2
- EP 0246069 B1
- WO 0210239 A
- DE 3915929 A1